# EUROPEAN PATENT APPLICATION

(11) **EP 1 548 124 A1**
(43) Date of publication of application: **29.06.2005**
(21) Application number: 02787326.4
(22) Date of filing: 10.12.2002
(51) Int. Cl.: C12Q 1/68, B08B 3/12

(54) **METHOD AND APPARATUS FOR WASHING SOLID SUBSTRATE WITH ULTRASONIC WAVE AFTER HYBRIDIZATION REACTION**

(30) Priority: 16.08.2002 CN 02128860
(71) Applicant: Tsinghua University, Beijing 100084 (CN); Capital Biochip Company Ltd, Haidian District, Beijing 100084 (CN)
(72) Inventor: LI, Gang, Beijing 100084 (CN); XING, Wanli, Beijing 100084 (CN); WU, Ruige, Beijing 100084 (CN); GUO, Min, Beijing 100084 (CN); CHENG, Jing, Beijing 100084 (CN)
(74) Representative: Duhme, Torsten
(86) International application number: PCT/CN2002/000877
(87) International publication number: WO 2004/016808

(57) **Abstract**

A method and apparatus for washing solid substrate with ultrasonic wave after hybridization reaction are provided. The method includes (1) putting the solid substrate into a container having a washing solution, on which the hybridization reaction has been completed, (2) using an ultrasonic generator to produce a certain strength of ultrasonic wave and transmit it into the washing solution, (3) washing the substrate by means of the cavitation effect being produced in the washing solution by the ultrasonic wave. Since the cavitation bubbles in the washing solution can produce intensive efflux and local microslipstream against the solid surface, evidently reducing the liquid surface tension and friction and enhance the liquid flow, non-specific adsorbate and deposit bound weakly on the solid substrate can be washed down rapidly.

## Description

### Cross-Reference to Related Applications

This application is a national phase application of PCT application PCT/CN2002/000877, filed on December 10, 2002, which claims priority to Chinese patent application Serial No. 02128860.7, filed August 16, 2002. The contents of the above referenced applications are incorporated by reference in their entireties.

### Field of the Invention

The present invention relates to a method and device for washing solid substrate after hybridization reaction using ultrasonic wave, especially relates to the method and device for washing biochips after hybridization reaction. The method and device can be used in clinical diagnosis, basic life science research, agriculture, environmental monitoring, and other fields.

### Background of the Invention

Nonspecific adsorption is very common during hybridization reactions, which makes it difficult to analyze the hybridization results in biological and medical applications. Thus, washing to get rid of nonspecific adsorption is one of the important steps during biological and medical sample analysis and detection. The conventional washing method is carried out by submerging the surface area of the solid substrate to be washed in a washing solution and washing the solid substrate through mechanical vibration. Although such method is simple, it is time consuming and inefficient, leaving high background signals and poor uniformity. Furthermore, because such washing device has moving part, it is difficult to adapt the device for integration and miniaturization.

### Brief Summary of the Invention

The purpose of the present invention is to shorten the washing time after hybridization reaction, increase the contrast between the hybridization and background signals, and provide a hybridization reaction washing device that is structurally simple, fast, inexpensive, easy to operate, suitable for automation, and applicable to microreaction systems.

The purpose of the present invention is achieved by the following technical scheme: a method for washing a hybridized solid substrate (i.e., a solid substrate on which a hybridization reaction has been completed) using ultrasonic wave, the method includes 1) placing the hybridized solid substrate in a container having a washing solution; 2) generating a certain strength of ultrasound with an ultrasonic wave generator and transmitting the ultrasound into the washing solution, wherein the washing of the solid substrate is facilitated by cavitation effects in the washing solution produced by the ultrasound.

The present invention further provides a device for carrying out the washing method described above. The device includes a container having a washing solution, a hybridized solid substrate placed in the washing solution, wherein at least one ultrasonic resonance component is placed upon, below, or around the hybridized solid substrate, and wherein the ultrasonic resonance component is connected to a resonance circuit.

When washing, first place the hybridized solid substrate in the washing solution, then activate the ultrasonic resonance circuit, which drives the ultrasonic resonance component to produce ultrasound. The washing is facilitated by the cavitation effect produced by the ultrasound transmitted to the solution. The cavitation bubbles in the washing solution can produce violent jet streams and local microslipstream against the solid surface, evidently reduce the liquid surface tension and friction, destroy the boundary layer at the solid-liquid interface, and enhance the liquid flow. Accordingly, nonspecific adsorption and deposit bound weakly on the solid substrate can be rapidly washed away.

### Brief Description of the Drawings

Figure 1 is a schematic diagram of an exemplary device of the present invention.

Figure 2 is the front view of an ultrasonic resonance component made of piezoelectric ceramic disk used in an example of the present invention.

Figure 3 is the back view of an ultrasonic resonance component made of piezoelectric ceramic disk used in an example of the present invention.

Figure 4 shows the comparison of hybridization fluorescent signals obtained according to an exemplary method of the present invention and those obtained according to the conventional mechanical vibration method.

Figure 5 shows the comparison of background fluorescent signals obtained according to an exemplary method of the present invention and those obtained according to the conventional mechanical vibration method.

### Detailed Description of the Invention

Some embodiments of the present invention are hereinafter described with reference to the drawings.

In some embodiments, the hybridized solid substrate is first placed in the washing solution, wherein the surface of the substrate that is to be washed is brought into contact with the washing solution. The ultrasonic resonance component can be in direct contact with the washing solution, or can be adhered to the outside wall of the solution container by adhesives such as epoxy adhesives. The ultrasonic resonance component can be positioned at the upper part, the lower part, or the peripheral of the container. The material for the ultrasonic resonance component can be piezoelectric ceramic or quartz material. The shape of the ultrasonic resonance component can be column, cone, cylinder, cube, or any other shape suitable for generating ultrasonic wave resonance. The size of the ultrasonic resonance component can be in a range of 10µm³ to 1000cm³. The frequency of the ultrasonic wave generated by the ultrasonic resonance component can be in a range of 20kHz to 100MHz. The power of the ultrasonic wave generator can be in a range of 1 W to 200 W, and the mode of generating ultrasonic wave can be continuous or intermittent.

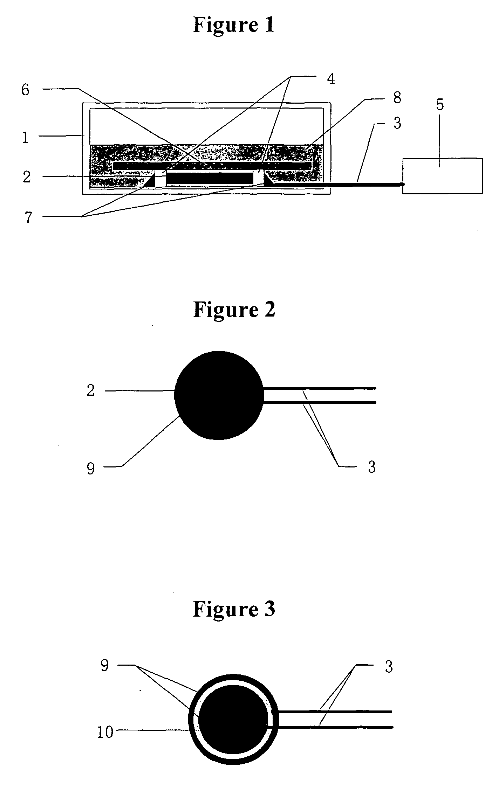

Figure 1 shows an actual example according to the present invention. This example provides a method of washing a hybridized gene chip using ultrasonic wave. A round piezoelectric ceramic disk 2 with diameter of 2.5cm and frequency of 1.7MHz was adhered to the inner side of plastic box 1 with the dimension of 8.5cm x 5cm x 2cm. The piezoelectric ceramic disk 2 was connected to ultrasonic resonance circuit 5 by wire 3 passing through a hole drilled at the side wall of the plastic box 1. The output power of ultrasonic resonance circuit 5 was 20W. The hole for wire passing was then sealed with epoxy adhesive. The outside rim of piezoelectric ceramic disk 2 was encircled by circular rubber sealing loop 4. The rubber sealing loop 4 was affixed to the bottom of plastic box 1 by epoxy adhesive 7, which was used to insulate the solder point from the washing solution to avoid short circuit. When washing, the gene chip to be washed 6 was submerged into washing solution 8 in plastic box 1.

Figures 2 and 3 show the arrangement of electrodes on the piezoelectric ceramic ultrasonic resonance component of the present invention. Figure 2 is the front view of the piezoelectric ceramic disk, and Figure 3 is the back view of the same: 9 represents the metal electrode coated on the surface of the ultrasonic piezoelectric ceramic disk, 3 represents the wire soldered to the ultrasonic piezoelectric ceramic disk, and 10 represents the exposed piezoelectric material at the back of the ultrasonic piezoelectric ceramic disk.

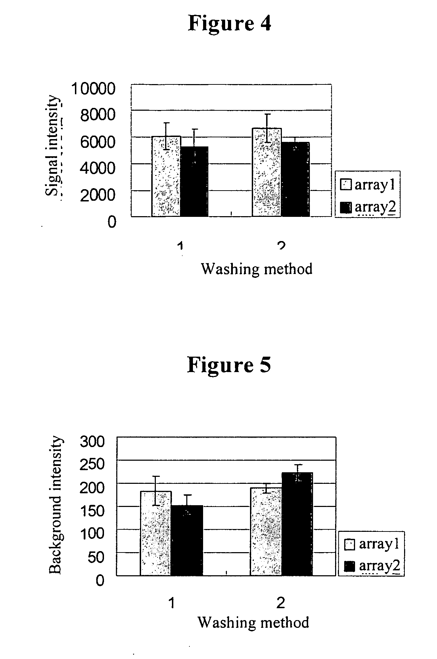

Figures 4 and 5 show the comparison of hybridization and background fluorescent signals obtained according to an exemplary method of the present invention and those obtained according to the conventional method. Washing condition 1 shown in figures 4 and 5 represents washing the liquid-solid hybridization system for 15 minutes by mechanical vibration (vibration frequency 80rpm, amplitude 15mm). Washing condition 2 represents washing the same system for 3 minutes using a 1.7MHz piezoelectric ceramic disk under the power of 20W. During washing, the gene chip was placed somewhere about 2mm above the piezoelectric ceramic disk, with its surface with probes on facing away from the ceramic disk. The washing solution was then added to submerge the gene chip. The washing solution was 0.1 % SDS, and its volume was 10 mL.

The probe, hybridization sample, and hybridization condition for Array 1 and Array 2 were the same. The probe was a 35mer oligonucleotide, which was attached covalently to an aldehyde modified glass slide. The target sample was a 1045 bp DNA, part of which matches perfectly with the probe. The sample DNA chain was labeled with Cy5 fluorescent molecules. The hybridization was carried out at 65 °C for 90 minutes. The detection was carried out using laser-induced fluorescence detection method. The instrument for detection was ScanArray 4000. The photomultiplier tube (PMT) was set at 75% of the maximum setting of the instrument. The power of the laser source was set at 80% of the maximum setting of the instrument.

As shown in Figures 4 and 5, the 3-minute wash with the help of ultrasound achieved the same washing results as the conventional washing method. Neither the hybridization fluorescent signals (Figure 4) nor the background fluorescent signals (Figure 5) showed obvious difference between the two methods. The experiment was repeated 8-9 times. These experiments demonstrate that the same washing results as the mechanical washing method can be achieved by utilizing the cavitation effect of ultrasound in a relatively shorter time. Accordingly, the detection process was accelerated.

### Industrial Applications

Compared with conventional washing techniques in hybridization reactions, the method of the present invention significantly shortens the washing time (the results of the example show that the washing time of the hybridized gene chip using the ultrasonic wave method was about one fifth of the time needed when using the conventional mechanical vibration method) and increases the contrast of the hybridization and background signals. Furthermore, the structure of the inventive device is simple (the core elements only include the ultrasonic resonance component, the corresponding ultrasonic resonance circuit, and the washing solution container) and has no moving parts, which makes it easier for integration and automation.

## Claims

1. A method for washing a solid substrate on which a hybridization reaction has been completed using ultrasonic wave, including 1) placing the solid substrate in a container having a washing solution; 2) generating certain strength of ultrasound with an ultrasonic wave generator and transmitting the ultrasound into the washing solution, wherein the washing of the solid substrate is facilitated by cavitation effect in the washing solution produced by the ultrasound.

2. The method of claim 1, wherein the power of the ultrasonic wave generator is in a range of about 0.1 W to about 200W.

3. The method of claim 1, wherein the ultrasonic wave frequency generated by the ultrasonic wave generator is in a range of about 20 kHz to about 100 MHz.

4. The method of claim 1, wherein the mode of generating ultrasonic wave by the ultrasonic wave generator is continuous or intermittent.

5. A device for washing a hybridized solid substrate according to the method of claim 1, comprising a container having a washing solution and a hybridized solid substrate placed in the solution, wherein at least one ultrasonic resonance component is placed upon, below, or around the hybridized solid substrate, and wherein the ultrasonic resonance component is connected with a resonance circuit.

6. The device of claim 6, wherein the resonance component is in direct or indirect contact with the washing solution.
